# EUROPÄISCHE PATENTSCHRIFT

(11) **EP 1 344 508 B1**
(45) Veröffentlichungstag und Bekanntmachung des Hinweises auf die Patenterteilung: **06.06.2007**
(21) Anmeldenummer: 02005632.1
(22) Anmeldetag: 12.03.2002
(51) Int. Cl.: A61F 2/44

(54) **Zwischenwirbelprothese, insbesondere für die Halswirbelsäule**
Intervertebral prosthesis especially for the cervical spine
Prothèse intervertébrale en particulier pour la colonne cervicale

(43) Veröffentlichungstag der Anmeldung: 17.09.2003
(73) Patentinhaber: Cervitech, Inc., Rockaway, NJ 07866 (US)
(72) Erfinder: Keller, Arnold, 23863 Kayhude (DE); McAfee, Paul C., M.D. Scoliosis and Spine Center, Baltimore , MD 21204 (US)
(74) Vertreter: Glawe, Delfs, Moll

(56) Entgegenhaltungen:
- WO-A-00/53127
- WO-A-01/01893
- WO-A-01/64142
- WO-A-99/65412
- FR-A- 2 718 635

## Beschreibung

Zwischenwirbelprothesen dienen dem Ersatz der Bandscheibe. Sie bestehen aus zwei Deckplatten, deren Außenflächen zur Verbindung mit benachbarten Wirbelkörpern ausgebildet sind, und einer von den Deckplatten eingeschlossenen Gelenkeinrichtung. Bei bekannten Prothesen (EP-B 471 821, WO 01/01893, FR 2718635) bildet die obere Deckplatte eine konkav-sphärische Gelenkfläche auf ihrer Innenseite, die mit der konvex-sphärischen Oberseite eines aus Polyethylen be stehenden Prothesenkerns zur Bildung eines Gelenks zusammenwirkt. Der Kern weist eine flache Unterseite und einen zylindrischen Rand auf, die passend in einem Sitz aufgenommen sind, der innenseitig von der unteren Deckplatte gebildet ist.

Bekannt ist ferner eine Zwischenwirbelprothese (WO 00/53127), die zwischen der unteren Deckplatte und dem Prothesenkern eine ebene Gleitfläche vorsieht, die dem Prothesenkern eine translatorische Bewegung gestattet, die in unterschiedlicher Weise begrenzt werden kann, beispielsweise auf eine Bewegung in AP-Richtung. Sie geht davon aus, daß die natürliche Bewegung der Wirbelsäule eine solche translatorische Komponente enthielte und diese daher auch in der prothetischen Nachbildung erwartet werden müßte.

Die Erfindung beruht auf der Erkenntnis, daß die Beobachtung zwar richtig ist, daß die Nachbildung der natürlichen Verhältnisse die Berücksichtigung einer translatorischen Bewegungskomponente verlangt, daß es aber in manchen Fällen im Interesse der Stabilität oder aus anderen Gründen notwendig sein kann, darauf zu verzichten. Sie schlägt daher vor, innerhalb eines und desselben Systems von Zwischenwirbelprothesen neben solchen Prothesen, deren Kern relativ zu der ihn haltenden Deckplatte mindestens in AP-Richtung beweglich ist, solche Typen mit übereinstimmender Außenform vorzusehen, die keine AP-Beweglichkeit zwischen dem Prothesenkern und der ersten Deckplatte aufweisen. Dem Arzt wird dadurch die Möglichkeit gegeben, intra-operativ zu entscheiden, welche der beiden Möglichkeiten er wählen will.

Der Kern muß sicher in der Prothese gehalten sein, damit er nicht etwa in den Rückenmarkskanal vordringt. Zu diesem Zweck können Bewegungsbegrenzungseinrichtungen an einer oder beiden Deckplatten vorgesehen sein, die die dem Kern gestattete Bewegungsstrecke eingrenzen. Diese können beispielsweise mit dem äußeren Rand des Kerns zusammenwirken. Beispielsweise kann die untere Deckplatte einen ganz oder teilweise umlaufenden, erhöhten Kragen aufweisen, der mit dem äußeren Rand des Kerns zusammenwirkt und so hoch ist, daß der Kern selbst bei einer gewissen Dehnung des wirbelszwischenraums nicht darüber hinweggleiten kann. Dieser Rand kann, wie es an sich bekannt ist (DE-C 30 23 353) auch stellenweise so erhöht werden, daß er Vorsprünge bildet, die in entsprechende Ausnehmungen der gegenüberliegenden Deckplatte eingreifen, um einen Käfig zur Zurückhaltung des Kerns zu bilden.

Die Beweglichkeit des Kerns gegenüber der ihn haltenden Deckplatte ist besonders wichtig in AP-Richtung, weil in der Sagittalebene die stärksten Relativbewegungen (Beugung und Streckung) stattfinden, während die seitlichen Biegungen vergleichsweise gering sind. Bei einer vorteilhaften Ausführungsform der Erfindung ist deshalb vorgesehen, daß die Bewegungsbegrenzungseinrichtung als Führungseinrichtung in AP-Richtung ausgebildet ist. Insbesondere kann sie von einander gegenüberliegenden, parallelen, seitlichen Führungsschienen gebildet sein, zwischen denen der Kern so gehalten ist, daß er sich lediglich in AP-Richtung bewegen kann. Dabei sind die Führungsschienen zweckmäßigerweise hinterschnitten, um mit einer in den Hinterschnitt eingreifenden Leiste des Kerns zusammenzuwirken. Auf diese Weise wird verhindert, daß der Kern sich von der ihn haltenden Deckplatte abhebt. Dies hat den Vorteil, daß die für die Eingrenzung der Kernbewegung vorgesehenen Einrichtungen nicht sehr hoch sein müssen und daher auch keine Gefahr besteht, daß sie die Relativbewegung der Deckplatten im Verhältnis zueinander behindern könnten. Damit der Kern nicht dorsal oder ventral aus dem Führungsbereich der Schienen herausgeleitet, können entsprechende Anschläge vorgesehen sein. Der dorsale Bewegungsanschlag ist zweckmäßigerweise starr mit der den Sitz (d.h. die Führungsschienen) bildenden Deckplatte verbunden. Auf der ventralen Seite sollte ein Bewegungsanschlag vorgesehen sein, der aus seiner Anschlagstellung entfernbar ist, damit man den Kern nach der Implantation der Deckplatte leichter einsetzen kann. Anschließend wird der Anschlag in der Stellung fixiert, in der er das Entweichen des Kerns verhindert.

Statt einer Bewegungsbegrenzungseinrichtung, die mit dem äußeren Rand des Kerns zusammenwirkt, kann auch eine solche vorgesehen sein, die mit einem inneren Rand einer Ausnehmung des Kerns zusammenwirkt. Beispielsweise kann der Kern auf der Seite, die er der ihn haltenden Deckplatte zuwendet, eine Ausnehmung aufweisen, die mit einem Vorsprung dieser Deckplatte zusammenwirkt. Die Ausnehmung kann eine in AP-Richtung langgestreckte Form aufweisen. In diesem Fall ist es zweckmä-ßig, das Gelenk um die vertikale Achse rotierbar zu gestalten. Wenn der Vorsprung in AP-Richtung kurz oder im Querschnitt kreisförmig begrenzt ist, so daß er sich im Verhältnis zu der Ausnehmung drehen kann, ist der Kern gegenüber der ihn haltenden Deckplatte in bezug auf die Vertikalachse drehbar, so daß auf eine Rotationsmöglichkeit des Prothesengelenks um diese Achse verzichtet werden kann. Man erwirbt dadurch größere Freiheit in der Gestaltung des Gelenks. Wenn es den Kern umdrehbar mit der oberen Deckplatte verbindet, wird die AP-Richtung des Kerns dabei durch die AP-Richtung der oberen Deckplatte vorgegeben.

Ein besonderer Aspekt des Erfindungsgedankens besteht darin, daß innerhalb eines Systems von Zwischenwirbelprothesen neben solchen, die die beschriebene AP-Beweglichkeit aufweisen, andere Typen mit übereinstimmender Außenform vorhanden sind, die keine AP-Beweglichkeit zwischen dem Prothesenkern und der ihn haltenden Deckplatte zeigen. Dies ermöglicht es dem Arzt, intraoperativ zu entscheiden, ob er AP-Beweglichkeit vorsehen will oder nicht. Zweckmäßigerweise sind die Deckplatten der in AP-Richtung beweglichen bzw. unbeweglichen Prothesen übereinstimmend geformt und ist lediglich der Kern unterschiedlich. Es kann aber auch vorgesehen sein, daß der Prothesenkern und die mit ihm das Gelenk bildende Deckplatte in allen Typen übereinstimmen, während die AP-Beweglichkeit durch Unterschiedlichkeit der den Prothesenkern haltenden Deckplatte begründet ist. Schließlich besteht auch die Möglichkeit, daß alle drei Komponenten übereinstimmen und lediglich derjenige Anschlag, der ventral den Weg des Prothesenkerns in AP-Richtung begrenzt, unterschiedlich lokalisiert ist.

Wenn oben von unterer und oberer Deckplatte die Rede ist, so soll damit nicht zum Ausdruck gebracht werden, daß die den Sitz für den Kern bildende Deckplatte stets unten angebracht sein müßte. Vielmehr kann die Anordnung auch umgekehrt gewählt werden. In den Ansprüchen wird deshalb allgemeiner von einer ersten und einer zweiten Deckplatte gesprochen.

Um daß Abheben des Kerns von der ihn haltenden Deckplatte zu verhindern, kann vorgesehen sein, daß der Vorsprung und die Ausnehmung zusammenwirkend hinterschnitten ausgebildet sind.

Bevorzugte Ausführungsformen werden im folgenden anhand der Zeichnungen erläutert. Darin zeigen:
- Fig. 1: einen Frontalschnitt,
- Fig. 2: einen Sagittalschnitt,
- Fig. 3: eine Zerlegungsdarstellung einer ersten Ausführungsform,
- Fig. 4: die Zerlegungsdarstellung einer zweiten Ausführungsform,
- Fig. 5: eine zu der Ausführungsform gemäß Fig. 4 gehörige Variante der unteren Deckplatte und
- Fig. 6: zwei Deckplatten einer weiteren Ausführungsform ohne Kern.

Die untere Deckplatte 1 und die obere Deckplatte 2 der ersten Ausführungsform weisen Oberflächen 3 bzw. 4 auf, die zur Verankerung am zugehörigen Wirbelkörper bestimmt sind. Sie sind vorzugsweise eben. Es sind aber auch andere im wesentlichen flache Gestaltungen einschließlich geeigneter Oberflächenstrukturen zur besseren Verankerung am Knochen denkbar. Die Deckplatten bestehen vorzugsweise aus Metall.

Die untere Deckplatte 1 wendet der oberen Deckplatte 2 eine ebene Oberseite 5 zu, die auf drei Seiten von einem Kragen 6 eingefaßt ist, der oberhalb eines inneren Hinterschnitts eine nach innen ragende Leiste 7 bildet. Die untere Deckplatte 1 ist in der Draufsicht etwa rechteckig gestaltet. Im Bereich ihrer Seiten 8, 9 verlaufen die dortigen Zweige des Kragens 6 zueinander parallel und geradlinig.

Die Oberfläche 5 und der Kragen 6 der unteren Deckplatte bilden einen Sitz für den Prothesenkern 10, der aus gleitgünstigem Werkstoff, beispielsweise Polyethylen, besteht. Er hat eine zur Fläche 5 passende ebene Unterfläche, die von einer Randleiste 11 umgrenzt wird, oberhalb welcher sich eine Nut 12 befindet. Die Leiste 11 greift in den Hinterschnitt des Kragens 6 unterhalb der Leiste 7. Die Leiste 7 greift in die Nut 12 ein. Zwischen dem Kragen 6 der unteren Deckplatte 1 und dem Rand des Kerns 10 ist Gleitspiel vorhanden.

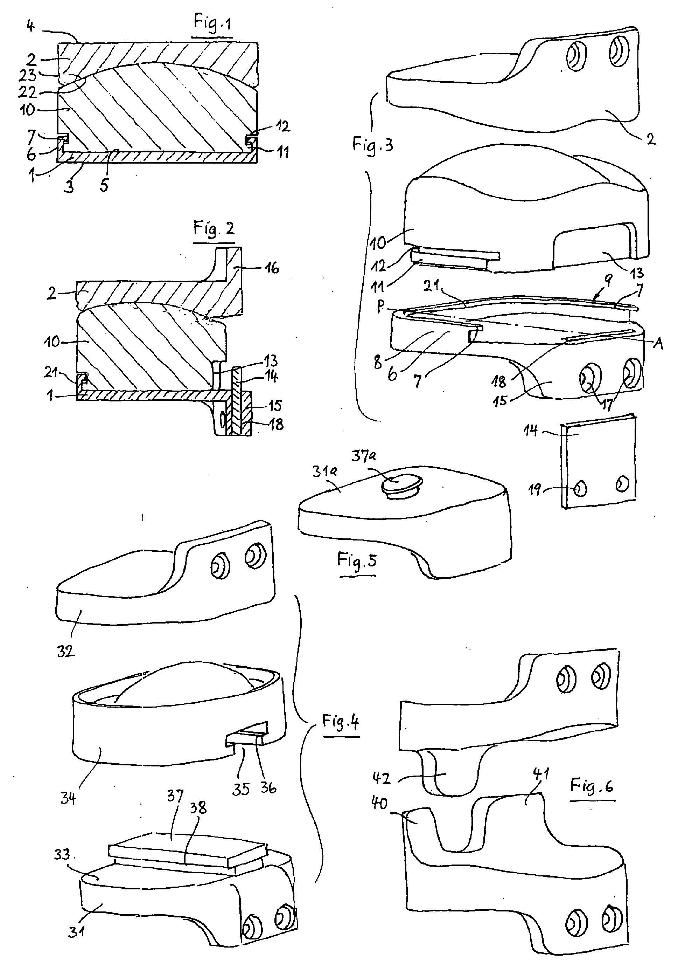

Der Kern 10 hat beidseitig lateral (in Fig. 1 rechts und links) und dorsal (in Fig. 2 links) dieselbe Umrißgestalt wie die untere Deckplatte 1. Die Form seiner Randleiste 11 und seiner Nut 12 folgt genau der Form der Kragens 6. Ventral (in Fig. 2 rechts) ist der Kern ein wenig kürzer als die untere Deckplatte, so daß zwischen ihrer ventralen Endfläche 13 und dem Anschlag 14 Spiel verbleibt.

An ihrem ventralen Rand weisen die Deckplatten 1, 2 je einen etwa rechtwinklig von ihnen abragenden Flansch 15, 16 auf, der Schraubenlöcher 17 zur Befestigung am Wirbelkörper enthält. Im Flansch 15 der unteren Deckplatte 1 befindet sich ein Schlitz 18, in welchem eine Anschlagplatte 14 verschiebbar gehalten ist. Sie kann die in Fig. 2 dargestellte Sperrstellung einnehmen, in welcher sie einen Anschlag für die nach vorne gerichtete Bewegung des Kerns 10 bildet. Sie kann in den Schlitz 18 auch soweit nach unten verschoben oder gänzlich daraus entfernt werden, daß der Prothesenkern leicht von ventral zwischen die Deckplatten eingebracht werden kann. Sie enthält zwei Bohrungen 19, die in der Sperrstellung der Platte 14 mit den Schraubenlöchern 17 fluchten. Wenn die untere Deckplatte 1 durch die Schraubenlöcher 17 am Wirbelkörper befestigt ist, gehen die Befestigungsschrauben auch durch die Löcher 19 und sichern dadurch die Platte 14 in ihrer Sperrstellung.

Die seitlichen Zweige des Kragens 6 bilden eine Führung für den Prothesenkern, in welcher dieser sich in der in Fig. 3 angedeuteten AP-Richtung um eine gewisse Strecke bewegen kann, nämlich um die Weite des Freiraums zwischen der ventralen Anschlagfläche 13 des Prothesenkerns und der Anschlagplatte 14. Der dorsale Teil 21 des Kragens 6 wirkt als Sicherungsanschlag, der ein Entweichen des Kerns in dorsaler Richtung aus dem Zwischenraum zwischen den Deckplatten 1 und 2 verhindert. Auf das Vorhandensein des Hinterschnitts an dem Kragen 6 und am Rand des Kerns 10 kommt es nur in den seitlichen Bereichen 8 und 9 der unteren Deckplatte 1 und des Kerns an, nicht aber im dorsalen Verlauf 21 des Kragens 6.

Oberseitig weist der Kern 10 eine vorzugsweise konvexsphärische Gelenkfläche 22 auf, die zur Bildung eines Gelenks zusammenwirkt mit der unterseitigen, konkav-sphärischen Gleitfläche 23 der oberen Deckplatte 2.

Bei der Beugebewegung schwenkt die obere Deckplatte 2 im Verhältnis zur unteren Deckplatte 1 in der Ansicht gemäß Fig. 2 ein wenig im Uhrzeigersinn und bei der Streckbewegung entgegengesetzt. Wenn die obere Deckplatte 2 dabei genau der durch die Gleitflächen 22, 23 vorgegebenen Richtung folgt, ist diese Schwenkbewegung mit einer translatorischen Bewegung verknüpft, die bei der Beugung nach vorne (in Fig. 2 nach rechts) und bei der Streckung nach hinten (in Fig. 2 nach links) gerichtet ist. Ein Teil dieser translatorischen Bewegung kann den physiologischen Gegebenheiten widersprechen und zu unerwünschten Spannungen führen. Diese Spannungen verursachen rückstellende Kräfte, die bei der erfindungsgemäßen Prothesengestaltung dazu führen, daß die obere Deckplatte sich mit dem relativ zur unteren Deckplatte entgegengesetzt bewegt und dadurch die unerwünschte Bewegungskomponente kompensiert.

Zwischen den zusammenwirkenden Führungseinrichtungen des Kerns und der unteren Deckplatte kann in seitlicher Richtung so viel Spiel gelassen werden, daß auch in dieser Richtung eine gewisse Relativbewegung möglich ist.

Das Ausmaß des Bewegungsspiels in AP-Richtung liegt vorzugsweise zwischen ein und vier, weiter vorzugsweise in der Größenordnung von zwei bis drei Millimetern. Falls eine Relativbeweglichkeit in seitlicher Richtung vorgesehen ist, sollte deren Ausmaß nicht über zwei Millimeter hinausgehen.

In der zweiten Ausführungsform gemäß Fig. 4 besteht die Prothese aus einer unteren Deckplatte 31 und einer oberen Deckplatte 32. Die untere Deckplatte weist eine obere, ebene Fläche 33 auf, auf der der Prothesenkern 34 aufliegt. Während dieser Kern in der ersten Ausführungsform an seinen Außenseiten geführt ist, besitzt er in der zweiten Ausführungsform eine Ausnehmung 35 mit hinterschnittenen Seitenrändern 36, die mit einem langgestreckten Vorsprung 37 der unteren Deckplatte mit entsprechend hinterschnittenen Rändern 38 zusammenwirken. Der Kern 34 wird dadurch - ebenso wie unter Bezugnahme auf das erste Ausführungsbeispiel erläutert - in AP-Richtung beweglich gegenüber der unteren Deckplatte 31 geführt. Außerdem ist er durch die zusammenwirkenden Hinterschnitte gegen ein Anheben von der unteren Deckplatte geschützt. Es können geeignete (nicht gezeigte) Anschläge vorgesehen sein, die das Herausrutschen des Prothesenkerns aus dem Plattenzwischenraum verhindern.

Die untere Deckplatte 31 kann durch die in Fig. 5 dargestellte untere Deckplatte 31a ersetzt werden, die sich von der unteren Deckplatte 31 dadurch unterscheidet, daß ihr Vorsprung 37a nicht langgestreckt, sondern in der Draufsicht kreisförmig begrenzt ausgeführt ist. Dies ermöglicht dem Prothesenkern 34, von dem angenommen wird, daß er in Bezug auf eine vertikale Achse mit der oberen Deckplatte 32 drehverbunden ist, eine Rotation um den Vorsprung 37a, ohne die gewünschte AP-Bewegung zu behindern. Dies kann bei asphärischer Ausbildung der Gleitflächen zwischen Kern und oberer Deckplatte erwünscht sein.

Eine solche Rotierbarkeit des Kerns gegenüber der unteren Deckplatte kann auch bei der Ausführungsform nach Fig. 1 bis 3 vorgesehen werden, indem der Rand 11, 12 des Kerns 10 kreisförmig gestaltet wird. Er kann sich dann nicht nur in AP-Richtung zwischen den parallelen, seitlichen Zweigen 8, 9 des Kragens 6 bewegen, sondern auch drehen. Statt dessen ist es auch möglich, sowohl den Rand des Prothesenkerns als auch den Kragen der unteren Deckplatte kreisförmig zu gestalten. Dann ist der Kern gegenüber der Deckplatte rotierbar, ohne eine Bewegbarkeit in AP-Richtung aufzuweisen.

Fig. 6 zeigt lediglich die Deckplatten eines weiteren Ausführungsbeispiels ohne den Prothesenkern und ohne die Einrichtungen, die diesem eine AP-Bewegung gegenüber der unteren Deckplatte ermöglichen. Es kommt hier lediglich auf die Demonstration einer Ausführungsform der Mittel an, die einem zwischen den Deckplatten gehaltenen Prothesenkern das Entweichen nach dorsal verwehren. Diese bestehen aus einer oder mehreren von der unteren Deckplatte hochragenden Zungen 40, 41 und einer oder mehreren von der oberen Deckplatte nach unten ragenden Zungen 42, die zueinander versetzt sind, so daß sie jeweils in den Zwischenraum zwischen oder neben den überliegenden Zungen eingreifen. Die Zungen sind in vertikaler Richtung so lang ausgebildet, daß sie selbst bei größtmöglicher Verschwenkung der Deckplatten sich nicht so weit voneinander entfernen, daß der Prothesenkern zwischen ihnen entweichen könnte. Entsprechende Einrichtungen können auch an den Seiten und ventral vorgesehen werden.

## Patentansprüche

1. System von Zwischenwirbelprothesen, insbesondere für die Halswirbelsäule, die im wesentlichen aus einer ersten, mit einem ersten Wirbelkörper zu verbindenden Deckplatte (1, 31), einer zweiten, mit dem zweiten Wirbelkörper zu verbindenden Deckplatte (2, 32) und einem Prothesenkern (10, 43) bestehen, der von einem Sitz der ersten Deckplatte gehalten ist und mit der zweiten Deckplatte ein Gelenk bildet, **dadurch gekennzeichnet, daß** das System neben Zwischenwirbelprothesentypen, in denen der Kern (10, 34) relativ zur ersten Deckplatte (1, 31) mindestens in AP-Richtung beweglich ist, solche Typen mit übereinstimmender Außenform umfaßt, die keine AP-Beweglichkeit zwischen dem Prothesenkern und der ersten Deckplatte aufweisen.

2. System nach Anspruch 1, **dadurch gekennzeichnet, daß** der Sitz eine mit einem Rand (11, 36) des Kerns (10, 34) zusammenwirkende Bewegungsbegrenzungseinrichtung aufweist, die als Führungseinrichtung in AP-Richtung ausgebildet ist.

3. System nach Anspruch 2, **dadurch gekennzeichnet, daß** die Führungseinrichtung von zwei gegenüberliegenden, parallelen seitlichen Führungsschienen (7) gebildet ist.

4. System nach Anspruch 3, **dadurch gekennzeichnet, daß** die seitlichen Führungsschienen (7) hinterschnitten sind und der Kern (10) eine in den Hinterschnitt eingreifende Leiste (11) aufweist.

5. System nach Anspruch 3 oder 4, **dadurch gekennzeichnet, daß** die erste Deckplatte (1) einen dorsalen Bewegungsanschlag (21) für den Kern (10) aufweist.

6. System nach einem der Ansprüche 3 bis 5, **dadurch gekennzeichnet, daß** die erste Deckplatte (1) einen aus seiner Anschlagstellung entfernbaren, ventralen Bewegungsanschlag (14) für den Kern (10) aufweist.

7. System nach Anspruch 2, **dadurch gekennzeichnet, daß** der Kern (34) eine Ausnehmung (35) aufweist, die mit einem Vorsprung (37, 37a) der ersten Deckplatte (31) zusammenwirkt.

8. System nach einem der Ansprüche 1 bis 7, **dadurch gekennzeichnet, daß** die Deckplatten der einander entsprechenden Typen mit bzw. ohne AP-Beweglichkeit übereinstimmen und der Kern unterschiedlich ist.

9. System nach einem der Ansprüche 1 bis 7, **dadurch gekennzeichnet, daß** die zweite Deckplatte und der Prothesenkern der einander entsprechenden Typen mit bzw. ohne AP-Beweglichkeit übereinstimmen und die erste Deckplatte unterschiedlich ist.

10. System nach einem der Ansprüche 1 bis 7, **dadurch gekennzeichnet, daß** alle drei Komponenten übereinstimmen und ein Anschlag, der die Beweglichkeit des Prothesenkerns ventral begrenzt, unterschiedlich ist.

## Claims

1. System of intervertebral prostheses, especially for the cervical spine, which principally comprise a first cover plate (1, 31) to be connected to a first vertebral body, a second cover plate (2, 32) to be connected to the second vertebral body, and a prosthesis core (10, 34) which is held by a seat of the first cover plate and forms a hinge with the second cover plate, **characterized in that**, in addition to intervertebral prosthesis types in which the core (10, 34) is movable at least in the AP direction relative to the first cover plate (1, 31), the system comprises types of corresponding external configuration that have no AP mobility between the prosthesis core and the first cover plate.

2. System according to Claim 1, **characterized in that** the seat has a movement-limiting device which interacts with an edge (11, 36) of the core (10, 34) and which is designed as a guide device in the AP direction.

3. System according to Claim 2, **characterized in that** the guide device is formed by two opposite and parallel lateral guide rails (7).

4. System according to Claim 3, **characterized in that** the lateral guide rails (7) are undercut, and the core (10) has a ledge (11) engaging in the undercut.

5. System according to Claim 3 or 4, **characterized in that** the first cover plate (1) has a dorsal limit stop (21) for the core (10).

6. System according to one of Claims 3 to 5, **characterized in that** the first cover plate (1) has a ventral limit stop (14) for the core (10), which ventral limit stop (14) can be removed from its limit stop position.

7. System according to Claim 2, **characterized in that** the core (34) has a recess (35) that interacts with a projection (37, 37a) of the first cover plate (31).

8. System according to one of Claims 1 to 7, **characterized in that** the cover plates of the matching types with or without AP mobility are identical, and the core is different.

9. System according to one of Claims 1 to 7, **characterized in that** the second cover plate and the prosthesis core of the matching types with or without AP mobility are identical, and the first cover plate is different.

10. System according to one of Claims 1 to 7, **characterized in that** all three components are identical, and a limit stop, which limits the mobility of the prosthesis core in the ventral direction, is different.

## Revendications

1. Système de prothèses intervertébrales, en particulier pour la colonne cervicale, qui se composent essentiellement d'une première plaque de recouvrement (1, 31) à assembler à un premier corps de vertèbre, d'une seconde plaque de recouvrement (2, 32) à assembler au second corps de vertèbre, et d'un noyau de prothèse (10, 34), qui est maintenu par un siège de la première plaque de recouvrement et qui forme une articulation avec la seconde plaque de recouvrement, **caractérisé en ce que** le système comprend, en plus de types de prothèses intervertébrales dans lesquels le noyau (10, 34) est mobile au moins dans la direction AP par rapport à la première plaque de recouvrement (1, 31), des types de forme extérieure correspondante, qui ne présentent aucune mobilité AP entre le noyau de prothèse et la première plaque de recouvrement.

2. Système selon la revendication 1, **caractérisé en ce que** le siège présente un dispositif de limitation de mouvement coopérant avec un bord (11, 36) du noyau (10, 34), qui est réalisé en forme de dispositif de guidage en direction AP.

3. Système selon la revendication 2, **caractérisé en ce que** le dispositif de guidage est formé par deux rails de guidage (7) latéraux, parallèles et se faisant face.

4. Système selon la revendication 3, **caractérisé en ce que** les rails de guidage latéraux (7) présentent une contre-dépouille et le noyau (10) comporte une nervure (11) s'engageant dans la contre-dépouille.

5. Système selon la revendication 3 ou 4, **caractérisé en ce que** la première plaque de recouvrement (1) comporte une butée de mouvement dorsale (21) pour le noyau (10).

6. Système selon l'une quelconque des revendications 3 à 5, **caractérisé en ce que** la première plaque de recouvrement (1) présente une butée de mouvement ventrale (14) pour le noyau (10), amovible de sa position de butée.

7. Système selon la revendication 2, **caractérisé en ce que** le noyau (34) présente une cavité (35), qui coopère avec une saillie (37, 37a) de la première plaque de recouvrement (31).

8. Système selon l'une quelconque des revendications 1 à 7, **caractérisé en ce que** les plaques de recouvrement des types se correspondant l'un à l'autre, avec ou sans mobilité AP, s'adaptent l'une à l'autre et le noyau est différent.

9. Système selon l'une quelconque des revendications 1 à 7, **caractérisé en ce que** la seconde plaque de recouvrement et le noyau de prothèse des types se correspondant l'un à l'autre, avec ou sans mobilité AP, s'adaptent l'un à l'autre et la première plaque de recouvrement est différente.

10. Système selon l'une quelconque des revendications 1 à 7, **caractérisé en ce que** les trois composants s'adaptent tous les uns aux autres et une butée ventrale, qui limite la mobilité du noyau de prothèse, est différente.
